# FASCICULE DE BREVET EUROPEEN

(11) **EP 3 119 403 B1**
(45) Date de publication et mention de la délivrance du brevet: **06.05.2020**
(21) Numéro de dépôt: 15715436.0
(22) Date de dépôt: 16.03.2015
(51) Int. Cl.: A61K 31/60, A61K 31/19, A61K 31/191, A61P 17/12

(54) **COMPOSITION À BASE D'ACIDES CARBOXYLIQUES ET SON APPLICATION POUR LE TRAITEMENT DES HYPERKÉRATOSES**
CARBOXYLSÄUREBASIERTE ZUSAMMENSETZUNG UND VERWENDUNG DAVON ZUR BEHANDLUNG VON HYPERKERATOSEN
CARBOXYLIC ACID-BASED COMPOSITION AND USE THEREOF FOR TREATING HYPERKERATOSES

(30) Priorité: 19.03.2014 FR 1452285
(43) Date de publication de la demande: 25.01.2017
(73) Titulaire: Lepeytre, Patrick, 83340 Le Luc en Provence (FR)
(72) Inventeur: Lepeytre, Patrick, 83340 Le Luc en Provence (FR)
(74) Mandataire: IPAZ
(86) Numéro de dépôt international: PCT/IB2015/051904
(87) Numéro de publication internationale: WO 2015/140690

(56) Documents cités:
- WO-A1-00/45808
- WO-A1-2013/180606
- DHAR S ET AL: "Treatment of warts with salicylic acid and lactic acid in flexible collodion wart pain", INDIAN JOURNAL OF DERMATOLOGY, VENEREOLOGY AND LEPROLOGY, MEDKNOW PUBLICATIONS ON BEHALF OF THE INDIAN ASSOCIATION OF DERMATOLOGISTS, VENEREOLOGISTS AND LEPROLOGISTS (IADVL), INDIA, vol. 60, no. 5, 1 janvier 1994 (1994-01-01), pages 286-289, XP009102520, ISSN: 0378-6323
- BHAT R M ET AL: "TOPICAL FORMIC ACID PUNCTURE TECHNIQUE FOR THE TREATMENT OF COMMON WARTS", INTERNATIONAL JOURNAL OF DERMATOLOGY, WILEY-BLACKWELL PUBLISHING LTD, UK, vol. 40, no. 6, 1 juin 2001 (2001-06-01), pages 415-419, XP001097776, ISSN: 0011-9059, DOI: 10.1046/J.1365-4362.2001.01242.X
- M. M. LIPKE: "An Armamentarium of Wart Treatments", CLINICAL MEDICINE & RESEARCH, vol. 4, no. 4, 1 décembre 2006 (2006-12-01), pages 273-293, XP055003664, ISSN: 1539-4182, DOI: 10.3121/cmr.4.4.273

## Description

L'invention a pour objet une composition à base d'acides carboxyliques pour le traitement chez l'Homme et l'animal des hyperkératoses virales ou non virales.

Elle concerne en particulier une composition pour le traitement de verrues, notamment de verrues profondes.

La verrue est liée à une infection virale à papillomavirus humains (HPV) au niveau épidermique. Plus de 60 sous-types ont été décrits, le type 1 correspondant aux verrues plantaires de type myrmécie, et le types 2 ou 4 aux verrues vulgaires.

Le caractère profond de la verrue plantaire, du fait de l'appui régulier du corps et de la chaleur confinée des voûtes plantaires, nécessite un temps de traitement devant correspondre au taux de renouvellement épidermique, soit 3 semaines environ, pour une action continue efficace et une bonne tolérance.

Le traitement des verrues, notamment des verrues plantaires, ne dispose pas actuellement de thérapeutique de référence. Toutes les études donnent un taux de guérison d'un maximum de 60% à 3 mois, quelle que soit la thérapeutique utilisée.

Les traitements locaux reposent fréquemment sur l'utilisation d'agents kératolytiques. Ainsi, l'association classique est celle de l'acide salicylique avec l'acide lactique, telle que décrite par exemple dans S. Dhar et al. (Ind J Dermatol Venereol Leprol 1994; 60: 286-9).

L'utilisation d'un seul acide est également courante : acide salicylique, acide trichloroacétique, ou acide formique.

L'efficacité de ces agents se révèle toutefois insuffisante pour les verrues profondes. La cryothérapie à l'azote liquide constitue une autre approche du traitement des verrues. Efficace sur les verrues superficielles, elle nécessite toutefois le recours à des séances multiples pour les verrues profondes, limitant ainsi son indication du fait de son caractère douloureux et de son coût.

Le recours à une chimiothérapie locale a également été proposé, mais son application est limitée par la douleur et la toxicité (risque de nécrose).

Une autre forme de traitement correspond à la chirurgie d'exérèse, notamment par l'emploi de laser C0₂.

Le caractère invalidant lié à cet acte limite cependant son champ d'application.

Les inconvénients ci-dessus ont conduit l'inventeur à rechercher de nouveaux moyens de traitement des verrues, en particulier des verrues profondes, et plus généralement de toute forme d'hyperkératoses. Les études effectuées ont montré qu'une grande efficacité et une bonne tolérance étaient obtenues en utilisant un mélange de 3 acides carboxyliques kératolytiques.

Ces résultats sont également observés pour des pathologies aboutissant de manière générale à une hyperkératinisation épidermique.

L'invention a donc pour but une nouvelle composition, à la fois économique et bien tolérée, à base d'acides carboxyliques à effet synergique pour le traitement d'hyperkératoses notamment de verrues, en particulier de verrues profondes.

Elle a également pour but de fournir une formulation pharmaceutique de grande efficacité pour traiter les hyperkératoses, notamment des verrues, et obtenir rapidement leur disparition.

L'invention porte ainsi sur une composition pharmaceutique à base d'acides carboxyliques, pour son utilisation dans le traitement d'hyperkératoses, caractérisé en ce qu'elle renferme un mélange, en quantité thérapeutiquement efficace, d'acide lactique, d'acide salicylique et d'acide formique en association avec un véhicule pharmaceutiquement acceptable.

Dans cette composition, chaque acide apporte son mode d'action, tout en conduisant à une synergie des effets obtenus.

On entend par effet synergique le fait que pour une concentration donnée de chacun des différents acides carboxyliques, la combinaison a un effet supérieur à celui observés lorsqu'ils sont utilisés à ladite concentration, seuls ou en combinaison de deux acides carboxyliques.

Cette association d'acides carboxyliques présente également l'avantage d'éviter une trop forte concentration en principes actifs pour une bonne tolérance du produit fini.

L'invention vise en particulier, à cet effet, une composition pharmaceutique caractérisée en ce que lesdits acides sont présents, par rapport au poids de la composition, à raison de

| | |
|---|---|
| acide lactique : | 10 à 20%, |
| acide salicylique : | 10 à 20% |
| acide formique : | 5 à 20% |

étant entendu que la somme des pourcentages partiels est au plus égale à 100.

De préférence, lesdits acides carboxyliques sont présents à raison de :

| | |
|---|---|
| acide lactique : | 17 à 18%, notamment de 17,3 à 17,5% |
| acide salicylique : | 13 à 14,5%, notamment de 13 à 13,5% |
| acide formique : | 11 à 12%, notamment de 11,5 à 12%. |

L'acide salicylique et l'acide lactique agissent notamment par dissolution du ciment intercellulaire et l'acide formique agit par dessiccation des tissus.

Avantageusement, la composition de l'invention se présente sous forme appropriée pour une application topique sur la partie à traiter, de préférence qui ne coule pas et sèche rapidement.

Une composition préférée comprend à cet effet, comme véhicule pharmaceutiquement acceptable, un ou plusieurs excipients choisis parmi des solvants tels que l'éthanol, des dérivés de glycérol, des cétones ou des éthers, renfermant de préférence des agents épaississants, comme des dérivés de cellulose, ou des agents filmogènes.

La composition de l'invention, telle que définie ci-dessus, permet de manière avantageuse un nombre d'applications compatible avec une activité quotidienne normale. Ainsi, l'invention fournit une composition qui s'avère efficace en 3 semaines avec une seule application par jour.

Un taux de guérison de 80% environ est obtenu dans le traitement des verrues plantaires de type myrmécie, les plus profondes et difficiles à traiter, ce qui confère un grand intérêt à la composition de l'invention.

Selon un autre aspect, l'invention vise l'utilisation d'une composition telle que définie ci-dessus pour la fabrication d'un médicament pour le traitement de verrues, en particulier de verrues profondes.

Selon un autre aspect, l'invention vise une composition pour utilisation dans une méthode de traitement de verrues, en particulier de verrues profondes, chez une personne qui en a besoin, ladite méthode comprenant l'application de la composition renfermant un mélange, en quantité thérapeutiquement efficace, d'acide lactique, d'acide salicylique et d'acide formique en association avec un véhicule pharmaceutiquement acceptable.

De même, la composition de l'invention présente une grande efficacité pour traiter toute pathologie conduisant à une hyperkératinisation épidermique, notamment les lésions virales ou encore les kératoses de pression comme les durillons, œil de perdrix et kératodermies localisées, par exemple les kératodermies ponctuées.

D'autres caractéristiques et avantages de l'invention sont donnés dans les exemples qui suivent avec référence aux Figures 1A à 1C qui représentent les résultats obtenus dans 3 traitements avec la composition de l'exemple :
- Figure 1A : sur dessous d'orteil à J=0 et J=21
- Figure 1B : sur voûte plantaire à J=0 et J=28
- Figure 1C : sur voûte plantaire à J=0 et J= 21
   et aux figures 2A à 2C qui représentent les résultats obtenus avec
- Figure 2A de l'acide formique seul : sur voûte plantaire à J=0 et J=15
- Figure 2B de l'acide salicylique et de l'acide lactique en mélange : sur voûte plantaire à J=0 et J=21
- Figure 2C, une association des 3 acides conforme à l'exemple : sur voûte plantaire à J=0 et J=20
Les formulations comprenant l'acide formique seul ou le mélange acide salicylique et de acide lactique sont préparées de telle sorte que les concentrations finales de ces composés sont identiques à celle de la formulation de l'exemple ci=dessus Exemple : Formulation pour traitement de verrues plantaires de type myrmécie

| | |
|---|---|
| - Acide lactique | 4,5 g correspondant à 17 % |
| - Acide salicylique | 3,5 g correspondant à 13,4 % |
| - Acide formique | 3 g correspondant à 11,5 % |
| - Collodion élastique | 15 g correspondant à 57,7 % |

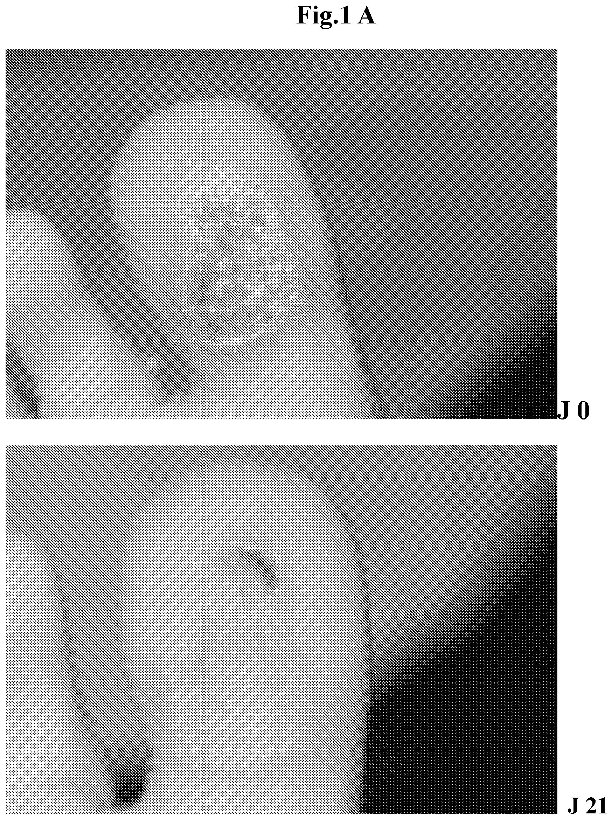

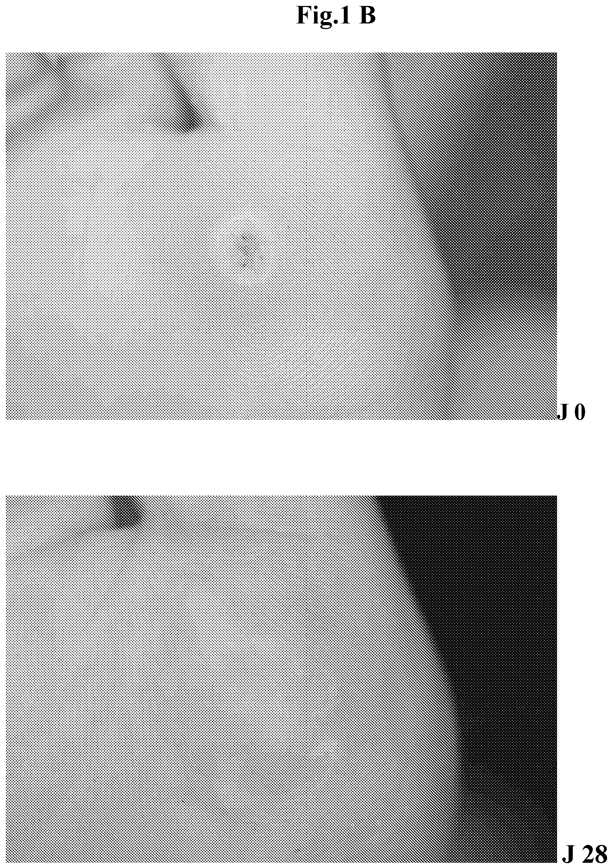

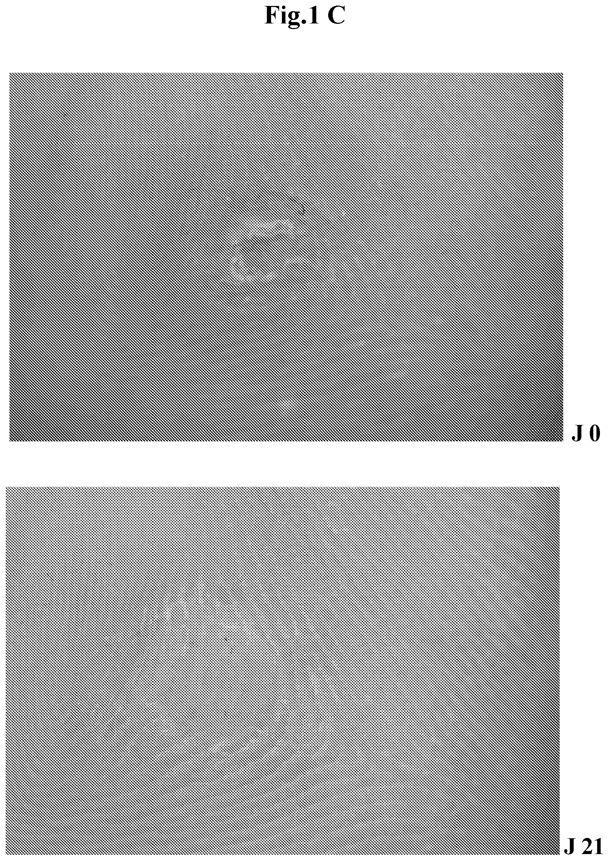

Comme le montrent les données sur les Figures 1A à 1C, pour 3 cas différents de verrues, une disparition totale est obtenue dès 21 jours de traitement.

L'invention fournit ainsi des moyens de grande efficacité pour le traitement des verrues profondes en une seule application par jour sur généralement 3 semaines, bien tolérés par le patient.

### Exemple comparatif :

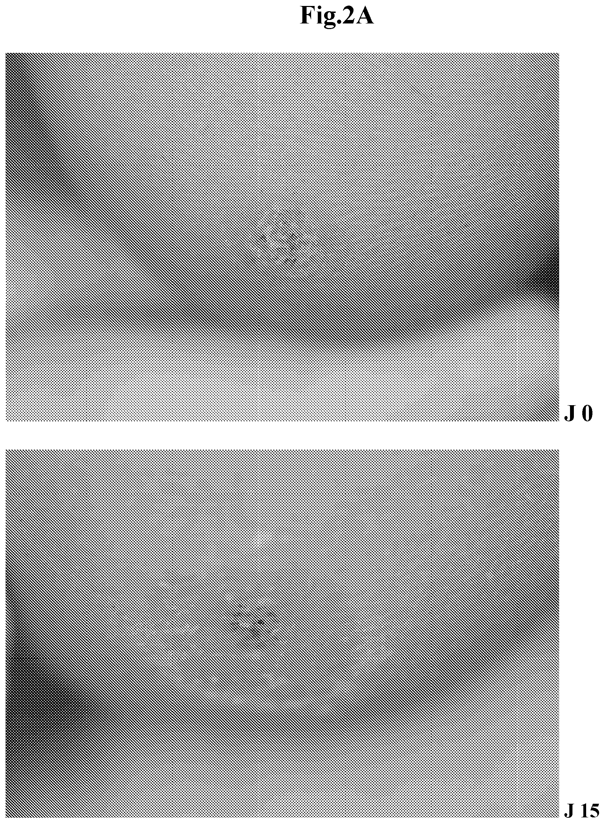

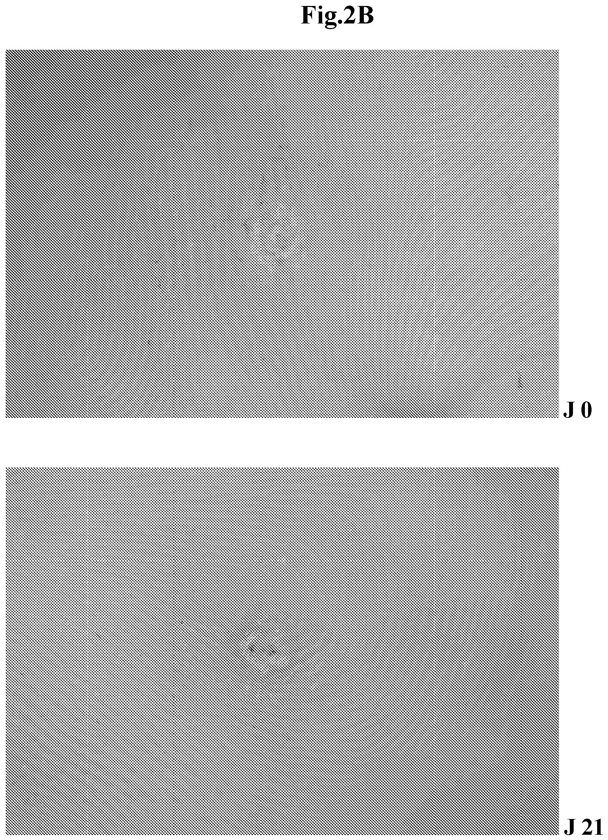

Pour vérifier l'effet synergique du mélange d'acides carboxyliques sur le traitement de verrues, 3 essais ont été réalisés, respectivement, avec de l'acide formique seul, un mélange d'acide salicylique avec de l'acide lactique et une formulation selon l'invention renfermant l'acide formique, l'acide salicylique et l'acide lactique.
Les résultats donnés sur les Figures 2A et 2B montrent clairement que le traitement n'est pas efficace en utilisant l'acide formique seul (la guérison ne dépasse pas 75%) ou le mélange acide salicylique + acide lactique (un décapage uniquement superficiel est obtenu, la guérison ne dépasse pas 40%).

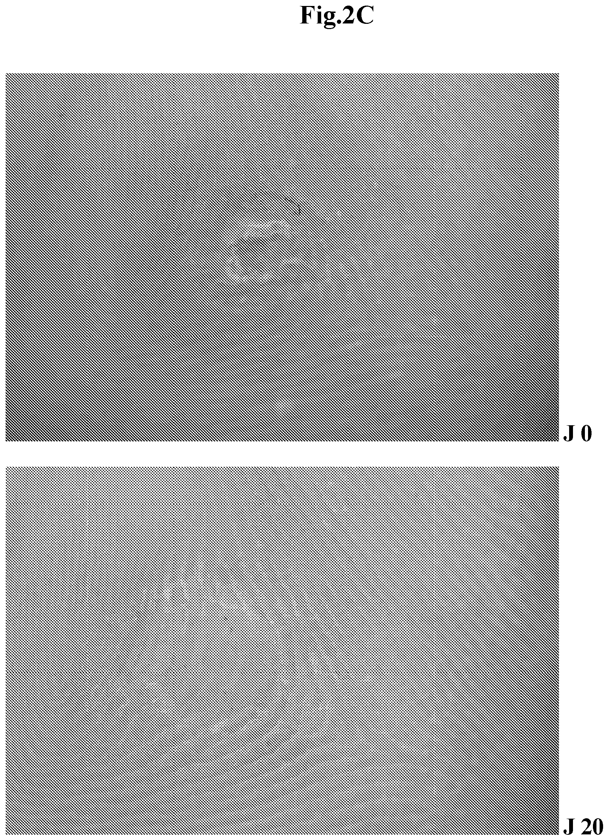

En revanche, comme le montre la Figure 2C, les verrues disparaissent totalement après 20 jours de traitement avec le mélange de l'invention, ce qui démontre l'effet synergique exercé par l'association des 3 acides.

## Revendications

1. Composition pharmaceutique à base d'acides carboxyliques, pour son utilisation dans le traitement des hyperkératoses, **caractérisée en ce qu'**elle renferme un mélange, en quantité thérapeutiquement efficace, d'acide lactique, d'acide salicylique et d'acide formique, en association avec un véhicule pharmaceutiquement acceptable.

2. Composition pour son utilisation selon la revendication 1, **caractérisée en ce que** lesdits acides sont présents, par rapport au poids de la composition, à raison de
| | |
|---|---|
| l'acide lactique : | 10 à 20% |
| l'acide salicylique : | 10 à 20% |
| l'acide formique : | 5 à 20% |
étant entendu que la somme des pourcentages partiels est au plus égale à 100.

3. Composition pour son utilisation selon la revendication 2, **caractérisée en ce que** lesdits acides sont présents, par rapport au poids de la composition, à raison de
| | |
|---|---|
| l'acide lactique : | 17,3 à 17,5% |
| l'acide salicylique : | 13 à 13,5% |
| l'acide formique : | 11,5 à 12% |

4. Composition pour son utilisation selon l'une quelconque des revendications 1 à 3 **caractérisée en ce qu'**elle se présente sous forme appropriée pour une application topique sur la partie à traiter, chez l'Homme ou l'animal.

5. Composition pour son utilisation selon la revendication 4, **caractérisée en ce qu'**elle comprend comme véhicule pharmaceutiquement acceptable, un ou plusieurs excipients choisis parmi des solvants tels que l'alcool éthylique, des dérivés de glycérol, des cétones ou des éthers, renfermant de préférence des agents épaississants comme des dérivés de cellulose ou des agents filmogènes.

6. Composition pour son utilisation selon l'une quelconque des revendications 1 à 5, **caractérisée en ce que** les hyperkératoses sont choisies parmi les verrues, en particulier les verrues profondes et les kératoses liées à des pathologies conduisant à une hyperkératinisation épidermique, notamment les lésions virales ou encore les kératoses de pression comme les durillons, œil de perdrix et kératodermies localisées, par exemple les kératodermies ponctuées.

## Patentansprüche

1. Pharmazeutische Zusammensetzung auf der Basis von Carbonsäuren zur Verwendung bei der Behandlung von Hyperkeratosen, **dadurch gekennzeichnet, dass** sie ein Gemisch von Milchsäure, Salicylsäure und Ameisensäure in therapeutisch wirksamer Menge in Verbindung mit einem pharmazeutisch annehmbaren Träger umfasst.

2. Zusammensetzung zur Verwendung gemäß Anspruch 1, **dadurch gekennzeichnet, dass** die Säuren in folgenden Anteilen vorhanden sind, bezogen auf das Gewicht der Zusammensetzung:
| | |
|---|---|
| Milchsäure | 10 bis 20% |
| Salicylsäure | 10 bis 20% |
| Ameisensäure: | 5 bis 20%; |
wobei die Summe der Teilprozentwerte höchstens gleich 100 sein soll.

3. Zusammensetzung zur Verwendung gemäß Anspruch 2, **dadurch gekennzeichnet, dass** die Säuren in folgenden Anteilen vorhanden sind, bezogen auf das Gewicht der Zusammensetzung:
| | |
|---|---|
| Milchsäure | 17,3 bis 17,5% |
| Salicylsäure | 13 bis 13,5% |
| Ameisensäure: | 11,5 bis 12%. |

4. Zusammensetzung zur Verwendung gemäß einem der Ansprüche 1 bis 3, **dadurch gekennzeichnet, dass** sie in geeigneter Form für eine topische Verabreichung auf dem zu behandelnden Teil beim Menschen oder bei einem Tier vorliegt.

5. Zusammensetzung zur Verwendung gemäß Anspruch 4, **dadurch gekennzeichnet, dass** sie als pharmazeutisch annehmbaren Träger einen oder mehrere Arzneimittelhilfsstoffe umfasst, die aus Lösungsmitteln, wie Ethylalkohol, Glycerinderivaten, Ketonen oder Ethern ausgewählt sind, wobei sie vorzugsweise Verdickungsmittel, wie Cellulosederivate oder Filmbildner, umfasst.

6. Zusammensetzung zur Verwendung gemäß einem der Ansprüche 1 bis 5, **dadurch gekennzeichnet, dass** die Hyperkeratosen aus Warzen, insbesondere tiefen Warzen, und Keratosen, die mit Erkrankungen verknüpft sind, welche zu einer Hyperkeratinisierung der Epidermis führen, insbesondere virale Läsionen, oder auch aus Druckkeratosen, wie Schwielen, Hühneraugen und lokalisierten Keratodermien, zum Beispiel punktuellen Keratodermien, ausgewählt sind.

## Claims

1. Pharmaceutical composition based on carboxylic acids, for its use in the treatment of hyperkeratoses, **characterized in that** it contains a mixture, in therapeutically effective amount, of lactic acid, salicylic acid and formic acid, in combination with a pharmaceutically acceptable vehicle.

2. Composition according to claim 1, **characterized in that** said acids are present, relative to the weight of the composition, at a rate of:
| | |
|---|---|
| lactic acid : | 10 à 20% |
| salicylic acid : | 10 a 20% |
| formic acid : | 5 à 20% |
it being understood that the sum of the partial percentages is at most equal to 100.

3. Composition according to claim 2, **characterized in that** said acids are present, relative to the weight of the composition, at a rate of:
| | |
|---|---|
| lactic acid : | 17,3 à 17,5% |
| salicylic acid : | 13 à 13,5% |
| formic acid : | 11,5 à 12% |

4. Composition according to any one of claims 1 to 3 **characterized in that** it is in suitable form for topical application on the part to be treated, in humans or animals.

5. Composition according to claim 4, **characterized in that** it comprises as a pharmaceutically acceptable vehicle, one or more excipients chosen from solvents such as ethyl alcohol, glycerol derivatives, ketones or ethers, preferably containing thickening agents such as cellulose derivatives or film-forming agents.

6. Use of a composition according to any one of claims 1 to 5, for the manufacture of a medicament for the treatment of warts, in particular of deep warts and of pathologies leading to epidermal hyperkeratinization, in particular viral lesions or also pressure keratoses such as calluses, partridge eye and localized keratoderma, for example punctate keratoderma.
